# EUROPEAN PATENT SPECIFICATION

(11) **EP 2 455 020 B1**
(45) Date of publication and mention of the grant of the patent: **02.09.2015**
(21) Application number: 11190051.0
(22) Date of filing: 22.11.2011
(51) Int. Cl.: A61B 17/34, A61B 17/02

(54) **Access assembly with anti-collapsing structure**
Zugangsanordnung mit einer einsturzvermeidenden Struktur
Ensemble d'accès doté d'une structure non pliable

(30) Priority: 23.11.2010 US 416505 P; 21.04.2011 US 201113091246
(43) Date of publication of application: 23.05.2012
(73) Proprietor: Covidien LP, Mansfield, MA 02048 (US)
(72) Inventor: Richard, Paul D., Shelton, Connecticut 06484 (US)
(74) Representative: Soames, Candida Jane

(56) References cited:
- EP-A1- 2 238 925
- EP-A1- 2 238 933
- WO-A1-2007/018458
- US-A- 4 984 564

## Description

### BACKGROUND

### Technical field

The present disclosure relates to a compressible access assembly for use in surgical procedures. More particularly, the present disclosure relates to compressible access assemblies including anti-collapsing structure.

### Background Of Related Art

Access assemblies configured for reception through an incision into an abdominal cavity are known, as are methods of inserting the access assemblies therethrough. Traditional access assemblies include a rigid cannula that is received through the tissue of the body wall into the body cavity. Endoscopic, laparoscopic and other suitable instruments may then be directed through a housing on the proximal end of the cannula to access the body cavity through the access assembly in a sealing manner.

Compressible assemblies configured for accessing a body cavity and permitting reception of instruments therethrough in sealing manner are also known. Such compressible assemblies are composed of silicone, thermoplastic elastomers (TPE), rubber, foam, gel and other compressible materials and are configured to be compressed to facilitate insertion into an incision. Typically, such assemblies are deformed by a surgeon using his/her fingers or with the PCL2\13647268\1 assistance of a grasping device, i.e., forceps. Compression of the assembly reduces the profile of the assembly, thereby facilitating reception of the assembly into the incision. Upon release of the compressive force, the compressed assembly returns to an uncompressed configuration. In the uncompressed configuration, the access assembly seals the incision into the body cavity. The assembly may have one or more access lumen for receiving instruments therethrough and may optionally be configured for connection with a source of insufflation gas.

Although configured to return to an uncompressed configuration once the compressive force is released, known compressible assemblies may fail to fully decompress. Additionally, known compressible assemblies may be subject to collapsing and/or excess flexion during use.

Therefore, it is desirable to provide a compressible access assembly which includes an anti-collapsing structure.

An access assembly as defined in the preamble of claim 1 is disclosed in WO 2007/018458.

### SUMMARY

An access assembly including an anti-collapsing structure is provided. The access assembly includes the features defined in claim 1.

In one embodiment, the anti-collapse structure includes one or more helical spring members. Alternatively, the anti-collapse structure may include a plurality of hoops. The anti-collapse structure may be selectively received within the compressible body. The compressible body may be formed about the anti-collapsing structure. The anti-collapsing structure may be formed of metal, plastic, polymer or a combination thereof. The anti-collapsing structure may be formed of a material having a circular, triangular, rectangular or oval cross-sectional profile.

The access assembly may include a lower rim at the distal end of the compressible body and an upper rim at the proximal end of the compressible body. The access assembly may also include a first spring section in the proximal end of the body and a second spring section in the distal end of the body. The compressible body may include three lumen. In one embodiment, the compressible body is composed of at least one of silicone, thermoplastic elastomers (TPE), rubber, foam, gel.

### DESCRIPTION OF THE DRAWINGS

Embodiments of a flexible access assembly are disclosed herein with reference to the drawings, wherein:
FIG. 1 is a perspective view of an embodiment of an access assembly according to the present disclosure;
FIG. 2 is an exploded side view of the access assembly of FIG. 1;
FIG. 3 is a cross-sectional side view of the access assembly of FIGS. 1 and 2;
FIG. 4A is a side view of an anti-collapsing structure according to an alternative embodiment of the present disclosure;
FIG. 4B is a side view of an anti-collapsing structure according to another embodiment of the present disclosure;
FIG. 5 is a cross-sectional side view of the access assembly of FIGS. 1-3 having a first end in a compressed condition;
FIG. 6 is a cross-sectional side view of the access assembly of FIG. 5, received through an incision in tissue;
FIG. 7 is an exploded perspective view of the access assembly of FIG. 6;
FIG. 8 is a cross-sectional side view of an access assembly according to another embodiment of the present disclosure; and
FIG. 9 is a cross-sectional side view of an access assembly according to still another embodiment of the present disclosure.

### DETAILED DESCRIPTION

Embodiments of the presently disclosed access assembly will now be described in detail with reference to the drawings wherein like numerals designate identical or corresponding elements in each of the several views. As is common in the art, the term "proximal" refers to that part or component closer to the user or operator, i.e. surgeon or physician, while the term "distal" refers to that part or component further away from the user. Although the access assemblies of the present disclosure will be described as relates to accessing an abdominal cavity through an incision in the abdominal wall, the access assemblies of the present disclosure may be modified for use in other closed procedures, i.e., laparoscopic, arthroscopic, endoscopic. Furthermore, the access assemblies of the present disclosure may be modified for use in accessing internal cavities through natural orifices, e.g., anus, vagina.

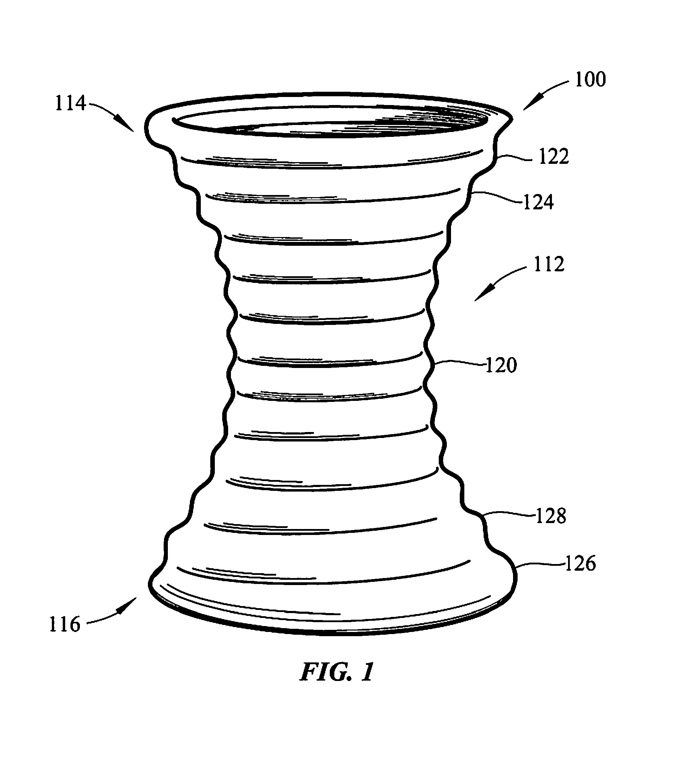

Referring initially to FIG. 1, an access assembly according to an embodiment of the present disclosure is shown generally as access assembly 100. Access assembly 100 is flexible and/or compressible to allow for insertion through a single incision in the body of a patient such that after insertion, access assembly 100 creates a seal within the incision through which a surgeon may insert and manipulate one or more surgical instruments to complete a procedure.

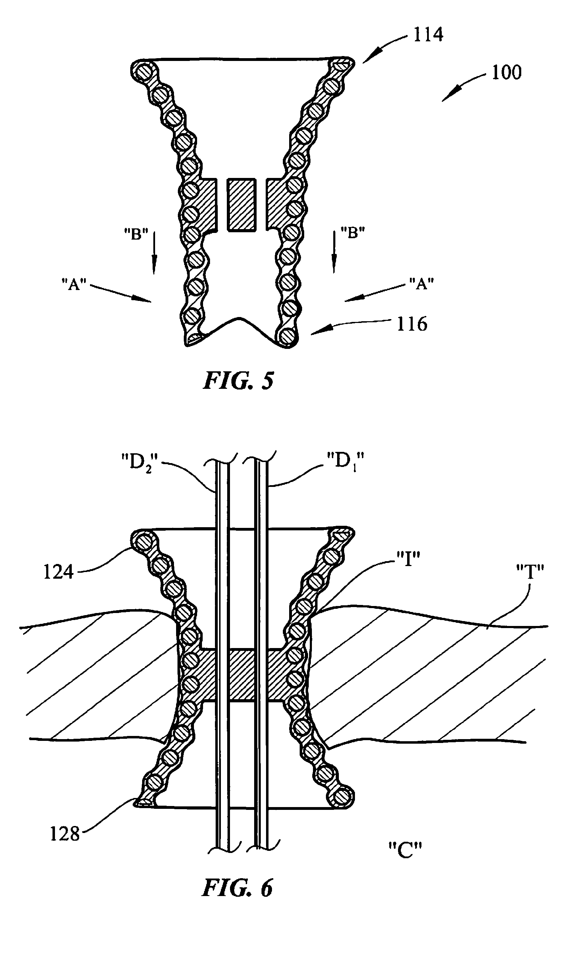

With reference to FIGS. 1-3, access assembly 100 includes a body 112 having substantially open proximal and distal ends 114, 116 and defines a substantially hourglass shape when viewed from the side. Body 112 may have a textured or contoured surface to facilitate engagement with tissue "T" (FIG. 6) when received through an incision "I" (FIG. 6) in tissue "T". Body 112 includes a central portion 120 having an upper rim 122 located at a proximal end 124 of central portion 120 and a lower rim 126 located at a distal end 128 of central portion 120. Central portion 120 is configured to span the thickness of tissue "T" (FIG. 6). Upper rim 122 and lower rim 126 aid in preventing movement of access assembly 100 longitudinally through incision "I" (FIG. 6). Upper and lower rims 122, 126 may have similar or different configurations. As the thickness of tissue "T" depends the body composition of the patient and the location through which the underlying cavity is being accessed, the length and size of access assembly 100 may be modified to suit a given procedure. As will be discussed in further detail below, the configuration of access assembly 100 including anti-collapsing structure 140 allows access assembly 100 to expand longitudinally along a length thereof.

Still referring to FIGS. 1-3, body 112 of access assembly 100 may be formed of various materials, such as, for example, silicone, thermoplastic elastomers (TPE), rubber, foam, gel, etc. In this manner, access assembly 100 may be compressed or squeezed prior to insertion through an incision "I" or natural orifice (not shown) in the body of a patient. In one embodiment, body 112 includes TPE material that is infused with an inert gas, e.g. CO₂ or Nitrogen, to form a foam structure. Body 112 may be coated with a lubricant, e.g. Parylene N or C, in order to create a lubricious surface. Various other coatings, e.g., hydrophilic, hydrophobic, bio-agents, anti-infection, analgesic, may also be employed to improve effectiveness of access assembly 100 or to adapt access assembly 100 for a specific procedure.

With reference still to FIGS. 1-3, as discussed above, proximal and distal ends 114, 116 of body 112 are substantially open to permit reception and manipulation of one or more surgical devices "D1", "D2" (FIG. 6) therethrough. Central portion 120 of body 112 defines a plurality of lumens 134, 136. As shown, access assembly 100 includes a pair of lumens 134, 136 having substantially similar size and shape for receiving instruments of substantially similar diameter. Alternatively, one or both of lumens 134, 136 may have various configurations and/or axial orientations for receiving instruments of various configurations in various orientations. In one embodiment, body 112 may define a single lumen for receiving a single, large instrument. Lumens 134, 136 extend through central portion 122 of body 112 and define longitudinal axes "x1", "x2" configured to receive surgical instruments "D1", "D2" (FIG. 6), cannula assemblies, valve assemblies and/or insufflation apparatus. Lumens 134, 136 may include a protective lining (not shown) extending the length thereof to prevent tearing of access assembly 100 as instruments are manipulated therethrough. Lumens 134, 136 may also be coated with a lubricant to assist in the insertion of surgical instruments "D1", "D2" therethrough. In one embodiment, each of lumens 134, 136 includes a valve member (not shown).

Still referring to FIGS. 1-3, access assembly 100 further includes anti-collapsing structure 140. Anti-collapsing structure 140 is configured to add structural support to access assembly 100. Anti-collapsing structure 140 may also be configured to permit longitudinal expansion of access assembly 100. In one embodiment, anti-collapsing structure 140 includes a single helical spring member 142 extending the length of body 112. Spring member 142 may be composed of metal, plastic, polymer, a combination thereof or any other flexible material. Although shown as having a circular cross-sectional profile, the wire forming spring member 142 may instead have rectangular, pentagonal, oval, triangular or other cross-sectional profiles. It is further envisioned that the size and/or cross-sectional profile of spring member 142 may vary along a length thereof. In this manner, different portions of body 112 will have different characteristics. Spring member 142 may be configured to increase hoop strength, to prevent collapse, to allow for longitudinal expansion and/or to assist in decompression of access assembly 100.

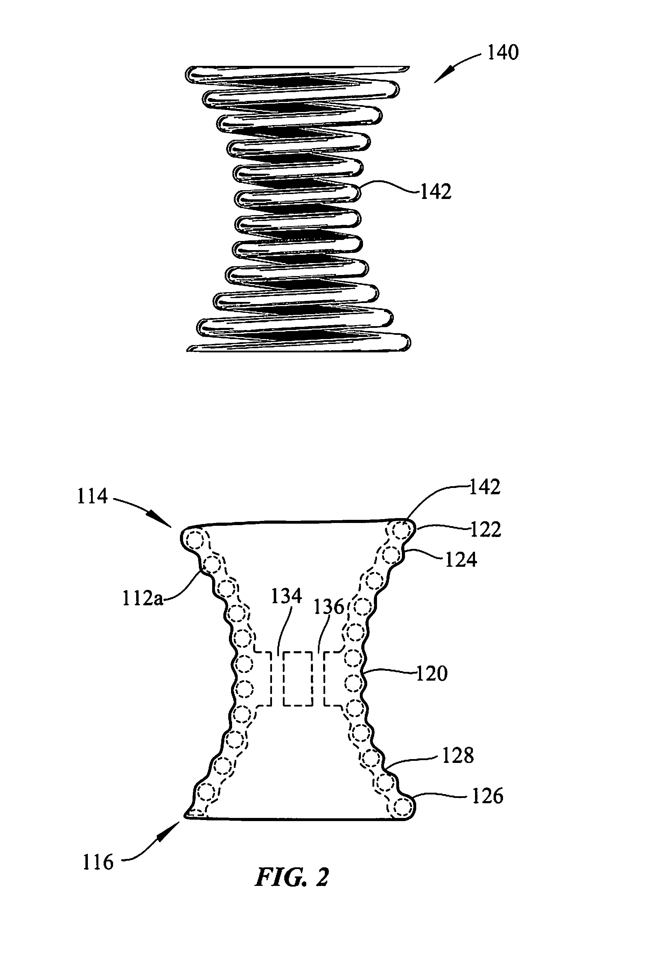

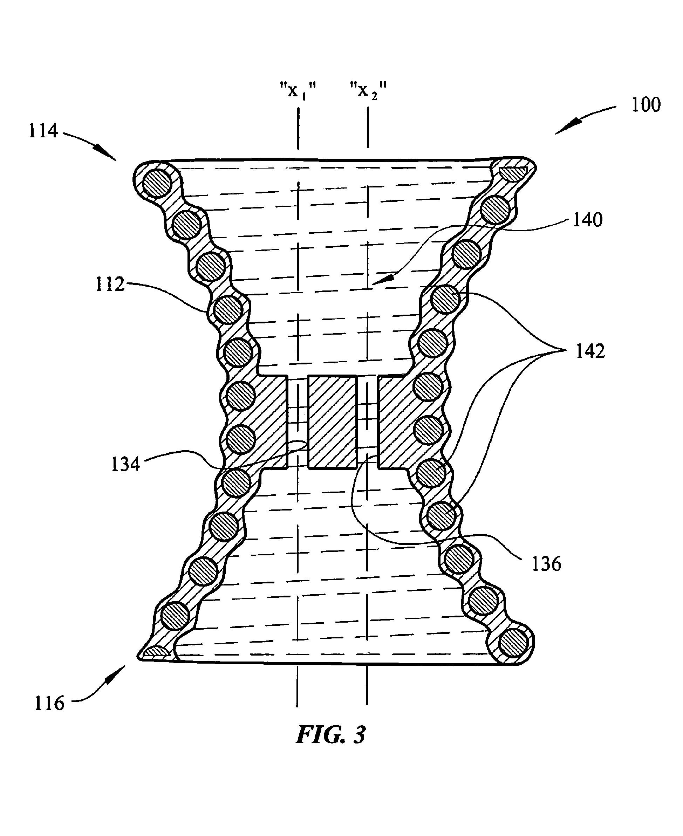

With particular reference to FIGS. 2 and 3, in one embodiment, body 112 of access assembly 100 is formed about spring member 142. In one embodiment, spring member 142 is placed in a mold (not shown) and a flexible material in liquid form is poured or injected into the mold about spring member 142 to create body 112. The flexible material is then allowed to gel/cure prior to removal from the mold. Alternatively, body 112 may be preformed. Preformed body 112 may include a spiral recess 112a (FIG. 2) to receive spring member 142. Alternatively, spring member 142 may include a piercing tip (not shown) that is configured to cut body 112 as spring member 142 is twisted therein. In yet another embodiment, body 112 may be divided into inner and outer portions (not shown). Spring member 142 may be received in between the inner and outer portions and the portions may be adhered, welded or otherwise secured to each other. In the embodiments where spring member 142 is inserted within body 112, a clinician may determine the characteristics of spring member 142, i.e., pitch, gage, size, width, etc., prior to or during a procedure and may customize access assembly 100 for the patient and procedure at hand.

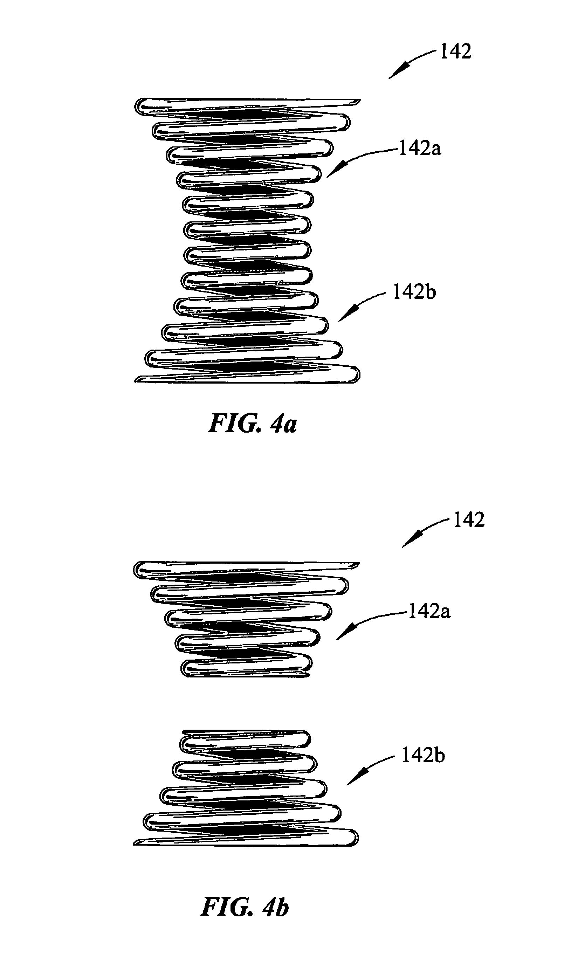

Turning briefly to FIG. 4A, in one embodiment, spring member 142 includes a spring having a first spring section 142a and a second spring section 142b. Either of first and second spring section 142a, 142b may correspond to either of proximal and distal ends 114, 116 of body 112. As shown, first spring section 142a is wider then second spring section 142b. In one embodiment, and as shown, first spring section 142a is also formed of a smaller gage wire than second spring section 142. Additionally, the pitch of first and second spring sections 142a, 142b may be the same or different. Further, first and second spring sections 142a, 142b may have the same or different lengths.

With reference now to FIG. 4B, in another embodiment, spring member 142 includes independent or separate first and second spring sections 142a, 142b. Although shown as including a space therebetween, it is envisioned that first and second spring sections 142a, 142b may be received within body 112 of access assembly 100 in contact with one another. Alternatively, a third spring section (not shown) may be received therebetween. In one embodiment, first spring section 142a is received in proximal end 114 of body 112, while second spring section 142b is received in distal end 116 of body 112. A third spring section (not shown) is optionally received within central portion 122 body 12. Alternatively, each of first and second spring sections 142a, 142b may extend into central portion 122. Because first and second spring sections 142a, 142b are not connected, each of proximal and distal ends 114, 116 of body 112 may be individually compressed without affecting the other of proximal and distal ends 114, 116. The absence of a spring section in either of proximal or distal ends 114, 116 and/or central portion 120 permits greater flexibility of that end or portion than if it including a spring section. As discussed above, first and second spring sections 142a, 142b may have the same or different size, pitch, length, cross-sectional profile, etc.

With reference to FIGS. 5 and 6, access assembly 100 is configured inserted and used in a traditional manner. Once an incision "I" is created through tissue "T", distal end 116 of body 112 is compressed to facilitate insertion of access assembly 100 therethrough. The flexibility of body 112 and spring member 142 permits the compression of distal end 116 of body 112, as indicated by arrows "A", such that access assembly 100 is configured to be inserted into incision "I". Although reference is made to "distal" end 116, in some embodiments, and as shown, proximal and distal ends 114, 116 of access assembly 100 are similar or substantially similar, as such, either of proximal or distal ends 114, 116 may be compressed and inserted through tissue "T". The configuration of body 112 and spring member 142 further permits the longitudinal expansion of access assembly 100, as indicated by arrows "B". In this manner, access assembly 100 may span tissue having a greater thickness. Once access assembly 100 is properly positioned within incision "I", release of the compressive force on distal end 114 of body 112 permits access assembly 100 to return to the uncompressed or expanded configuration (FIG. 5). In this manner, access assembly 100 creates an opening through tissue "T" to permit sealed reception of one or more surgical devices "D1", "D2" through tissue "T" and into body cavity "C". While positioned through incision "I" in tissue "T", access assembly 100 may be used to complete any number of procedures. Upon completion of a procedure, flexible access assembly 100 is removed from within incision "I" through tissue "T" by compressing distal end 116 of body 112 and retracting body 112 therefrom. Incision "I" is closed in a conventional manner.

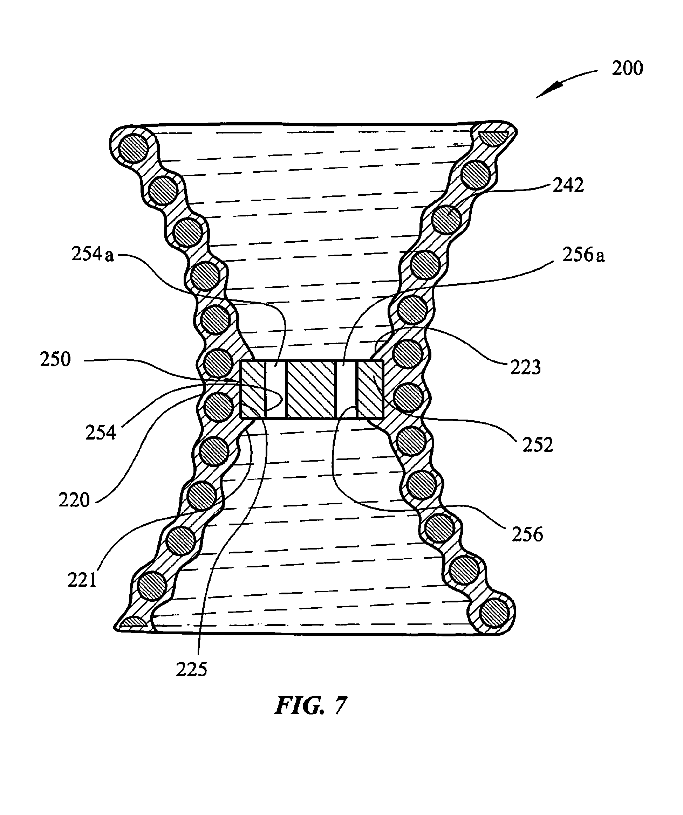

Turning now to FIG. 7, an alternative embodiment of an access assembly according to the present disclosure is shown generally as access assembly 200. Access assembly 200 is substantially similar to access assembly 100 described hereinabove, and will only be described as relates to the differences therebetween. Access assembly 200 includes a body 212 and a spring member 142. Spring member 142 is formed of a wire having a substantial square cross-sectional profile. Body 212 includes a central portion 220 having first and second flanges 221, 223 formed about a recess 225. Recess 225 is configured to receive a valve member 250. In one embodiment, valve member 250 includes a substantially disk-shaped member 252 defining one or more lumens 254, 256 therethrough. Disk member 252 may further include one or more valves 234a, 236a corresponding to lumens 254, 256, respectively. Recess 225 and disk member 252 are configured such that disk member 252 is rotatable relative to recess 225 while maintaining a sealed relationship therebetween. Either of flanges 221, 223 may be configured such that disk member 252 is selectively removable from recess 225. In this manner, disk member 252 may be changed throughout a procedure to provide access assembly 100 with different lumen and seal configurations. Disk member 252 may also be removed during insertion and removal of access assembly 200 to facilitate compression of access assembly 200.

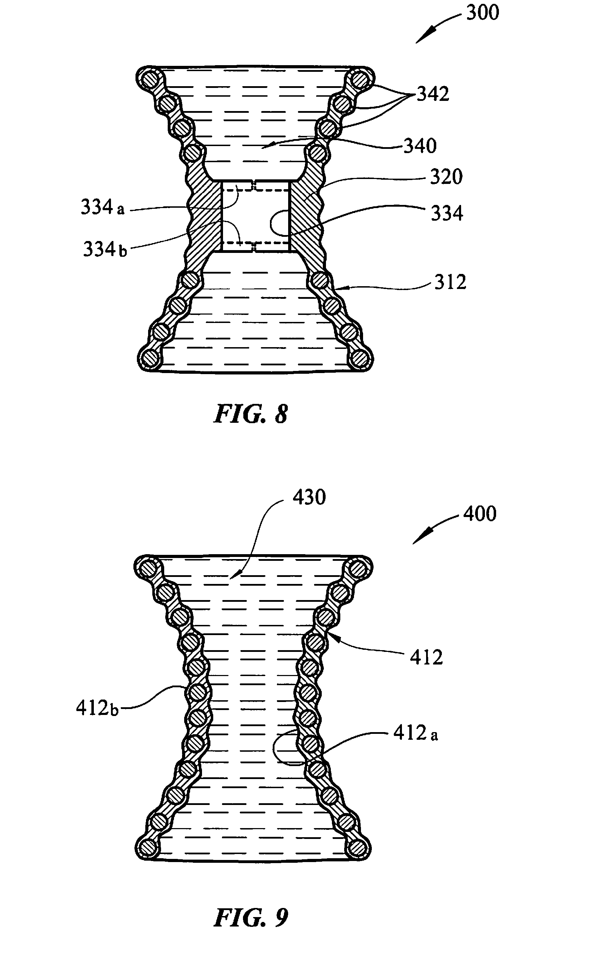

With reference now to FIG. 8, another embodiment of an access assembly according to the present disclosure is shown generally as access assembly 300. Access assembly 300 is substantially similar to access assemblies 100, 200 described hereinabove, and will only be described as relates to the differences therebetween. Access assembly 300 includes body 312 having a central portion 320 that defines a single lumen 334. Lumen 334 may include first and second valve members 334a, 334b. Access assembly 300 includes an anti-collapsing structure 340. Anti-collapsing structure 340 includes a plurality of hoops or rings 342. Hoops 342 may be formed of metal, plastic, polymer, a combination thereof or any other flexible material. Although shown as having a circular cross-sectional profile, the wire forming hoops 342 may instead have rectangular, pentagonal, oval, triangular or other cross-sectional profiles. It is further envisioned that the size and/or cross-sectional profile of hoops 342 may vary along a length of body 312. Hoops 342 may form continuous circles or instead may be broken. It is envisioned that body 312 may be formed about hoops 342, or that hoops 342 may be inserted into a preformed body 312.

Referring now to FIG. 9, yet another embodiment of an access assembly according to the present disclosure is shown generally as access assembly 400. Access assembly 400 is substantially similar to access assembly 200 described hereinabove, and will only be described as relates to the differences therebetween. Access assembly 400 includes a body 412 defining a central passageway 430 therethrough. Although shown having a contoured outer and inner surfaces 412a, 412b, either or both of outer and inner surface 412a, 412b of body 412 may be smooth.

It will be understood that various modifications may be made to the embodiments disclosed herein. For example, as noted hereinabove, the disclosed flexible access assemblies may be provided with multiple lumens in excess of the disclosed number of lumen. Additionally, the diameters or configuration of the disclosed lumen need not be identical but may be varied depending upon the contemplated surgical instruments to be utilized therethrough. Therefore, the above description should not be construed as limiting, but merely as exemplifications of particular embodiments. Those skilled in the art will envision other modifications within the scope of the claims appended hereto.

## Claims

1. An access assembly (100; 200; 300; 400) comprising:
a compressible body (112; 212; 312; 412) having proximal and distal ends (114, 116) and a central portion (120; 220; 320) extending there between, and
an anti-collapsing structure (140; 340) extending at least partially between the proximal and distal ends (114, 116) of the compressible body (112; 212; 312; 412), the anti-collapsing structure (140; 340) being configured to add structural support to the compressible body (112; 212; 312; 412) at least at each of the proximal and distal ends (114, 116) of the compressible body (112; 212; 312; 412) and being configured to permit compression of the compressible body (112; 212; 312; 412) during insertion into an incision and return the compressible body (112; 212; 312; 412) to an uncompressed condition following insertion, **characterised in that** the central portion (120;220;320) defines at least a first lumen configured to receive an instrument in a sealing manner.

2. The access assembly (100; 200; 400) of claim 1, wherein the anti-collapsing structure (140; 340) includes one or more helical spring members (142; 242).

3. The access assembly (100) of claim 1 or claim 2, wherein the anti-collapsing structure (140) includes a first spring section (142a) in the proximal end (114) of the body (112) and a second spring section (142b) in the distal end (116) of the body (112).

4. The access assembly (300) of claim 1, wherein the anti-collapsing structure (340) includes a plurality of hoops (342).

5. The access assembly (100; 200; 300; 400) of any preceding claim, wherein the anti-collapsing structure (140; 340) is selectively received within the compressible body (112; 212; 312; 412).

6. The access assembly of any preceding claim, wherein the compressible body is formed about the anti-collapsing structure.

7. The access assembly (100; 200; 300; 400) of any preceding claim, including a lower rim (126) at the distal end (116) of the compressible body and an upper rim (122) at the proximal end (114) of the compressible body (112; 212; 312; 412).

8. The access assembly (100; 200; 300; 400) of any preceding claim, wherein the compressible body (112; 212; 312; 412;) includes three lumens.

9. The access assembly (100; 200; 300; 400) of any preceding claim, wherein the compressible body (112; 212; 312; 412) is composed of at least one of silicone, thermoplastic elastomers (TPE), rubber, foam, gel.

10. The access assembly (100; 200; 300; 400) of any preceding claim, wherein the anti-collapsing structure (140; 340) is formed of metal, plastic, polymer or a combination thereof.

11. The access assembly (100; 200; 300; 400) of any preceding claim, wherein the anti-collapsing structure (140; 340) is formed of a material having a circular, triangular, rectangular or oval cross-sectional profile.

## Patentansprüche

1. Zugangsanordnung (100; 200; 300; 400), umfassend:
einen zusammendrückbaren Körper (112; 212; 312; 412) mit proximalen und distalen Enden (114, 116) und einem zentralen Abschnitt (120; 220; 320) der sich dazwischen erstreckt, und
eine nicht kollabierende Struktur (140; 340), die sich mindestens teilweise zwischen den proximalen und distalen Enden (114, 116) des zusammendrückbaren Körpers (112; 212; 312; 412) erstreckt, wobei die nicht kollabierende Struktur (140; 340) konfiguriert ist, um dem zusammendrückbaren Körper (112; 212; 312; 412) mindestens an jedem der proximalen und distalen Enden (114, 116) des zusammendrückbaren Körpers (112; 212; 312; 412) strukturelle Abstützung hinzuzufügen und konfiguriert ist, das Zusammendrücken des zusammendrückbaren Körpers (112; 212; 312; 412) während des Einsetzens in einen Einschnitt zu ermöglichen und den zusammendrückbaren Körper (112; 212; 312; 412) in einen nicht zusammengedrückten Zustand nach dem Einsetzen zurückzuführen, **dadurch gekennzeichnet, dass** der zentrale Abschnitt (120; 220; 320) mindestens ein erstes Lumen definiert, das konfiguriert ist, um ein Instrument in einer abdichtenden Weise aufzunehmen.

2. Zugangsanordnung (100; 200; 400) nach Anspruch 1, wobei die nicht-kollabierende Struktur (140; 340) eine oder mehrere spiralförmige Federelemente (142; 242) aufweist.

3. Zugangsanordnung (100) nach Anspruch 1 oder Anspruch 2, wobei die nicht kollabierende Struktur (140) einen ersten Federabschnitt (142a) in dem proximalen Ende (114) des Körpers (112) und einen zweiten Federabschnitt (142b) in dem distalen Ende (116) des Körpers (112) aufweist.

4. Zugangsanordnung (300) nach Anspruch 1, wobei die nicht kollabierende Struktur (340) mehrere Reifen (342) aufweist.

5. Zugangsanordnung (100; 200; 300; 400) nach einem der vorhergehenden Ansprüche, wobei die nicht kollabierende Struktur (140; 340) selektiv in dem zusammendrückbaren Körper (112; 212; 312; 412) aufgenommen ist.

6. Zugangsanordnung nach einem der vorhergehenden Ansprüche, wobei der zusammendrückbare Körper um die nicht kollabierende Struktur herum gebildet ist.

7. Zugangsanordnung (100; 200; 300; 400) nach einem der vorhergehenden Ansprüche, umfassend einen unteren Rand (126) an dem distalen Ende (116) des zusammendrückbaren Körpers und einen oberen Rand (122) an dem proximalen Ende (114) des zusammendrückbaren Körpers (112; 212; 312; 412).

8. Zugangsanordnung (100; 200; 300; 400) nach einem der vorhergehenden Ansprüche, wobei der zusammendrückbare Körper (112; 212; 312; 412) drei Lumen aufweist.

9. Zugangsanordnung (100; 200; 300; 400) nach einem der vorhergehenden Ansprüche, wobei der zusammenpressbare Körper (112; 212; 312; 412) aus mindestens einem aus Silikon, thermoplastischen Elastomeren (TPE), Gummi, Schaum, Gel zusammengesetzt ist.

10. Zugangsanordnung (100; 200; 300; 400) nach einem der vorhergehenden Ansprüche, wobei die nicht kollabierende Struktur (140; 340) aus Metall, Kunststoff, Polymer oder einer Kombination davon gebildet ist.

11. Zugangsanordnung (100; 200; 300; 400) nach einem der vorhergehenden Ansprüche, wobei die nicht kollabierende Struktur (140; 340) aus einem Material mit einem kreisförmigen, dreieckigen, rechteckigen oder ovalen Querschnittsprofil gebildet ist.

## Revendications

1. Ensemble d'accès (100 ; 200 ; 300 ; 400) comprenant :
un corps compressible (112 ; 212 ; 312 ; 412) ayant des extrémités proximales et distales (114, 116) et une partie centrale (120 ; 220 ; 320) s'étendant entre elles, et
une structure anti-effondrement (140 ; 340) s'étendant au moins partiellement entre les extrémités proximales et distales (114, 116) du corps compressible (112 ; 212 ; 312 ; 412), la structure anti-effondrement (140 ; 340) étant configurée pour ajouter un support structurel au corps compressible (112 ; 212 ; 312 ; 412) au moins au niveau de chacune des extrémités proximales et distales (114, 116) du corps compressible (112 ; 212 ; 312 ; 412) et étant configurée pour permettre la compression du corps compressible (112 ; 212 ; 312 ; 412) pendant l'insertion dans une incision et le retour du corps compressible (112 ; 212 ; 312 ; 412) à un état non comprimé après insertion, **caractérisé en ce que** la partie centrale (120 ; 220 ; 320) définit au moins une première lumière configurée pour recevoir un instrument d'une façon étanche.

2. Ensemble d'accès (100 ; 200 ; 400) selon la revendication 1, dans lequel la structure anti-effondrement (140 ; 340) inclut un ou plusieurs éléments formant ressorts à boudin (142 ; 242).

3. Ensemble d'accès (100) selon la revendication 1 ou la revendication 2, dans lequel la structure anti-effondrement (140) inclut une première section de ressort (142a) dans l'extrémité proximale (114) du corps (112) et une seconde section de ressort (142b) dans l'extrémité distale (116) du corps (112).

4. Ensemble d'accès (300) selon la revendication 1, dans lequel la structure anti-effondrement (340) inclut une pluralité de cercles (342).

5. Ensemble d'accès (100 ; 200 ; 300 ; 400) selon n'importe quelle revendication précédente, dans lequel la structure anti-effondrement (140 ; 340) est reçue de manière sélective dans le corps compressible (112 ; 212 ; 312 ; 412).

6. Ensemble d'accès selon n'importe quelle revendication précédente, dans lequel le corps compressible est formé autour de la structure anti-effondrement.

7. Ensemble d'accès (100 ; 200 ; 300 ; 400) selon n'importe quelle revendication précédente, incluant un rebord inférieur (126) au niveau de l'extrémité distale (116) du corps compressible et un rebord supérieur (122) au niveau de l'extrémité proximale (114) du corps compressible (112 ; 212 ; 312 ; 412).

8. Ensemble d'accès (100 ; 200 ; 300 ; 400) selon n'importe quelle revendication précédente, dans lequel le corps compressible (112 ; 212 ; 312 ; 412) inclut trois lumières.

9. Ensemble d'accès (100 ; 200 ; 300 ; 400) selon n'importe quelle revendication précédente, dans lequel le corps compressible (112 ; 212 ; 312 ; 412) est composé d'au moins un de silicone, d'élastomères thermoplastiques (TPE), de caoutchouc, de mousse, de gel.

10. Ensemble d'accès (100 ; 200 ; 300 ; 400) selon n'importe quelle revendication précédente, dans lequel la structure anti-effondrement (140 ; 340) est formée de métal, de plastique, de polymère ou d'une combinaison de ceux-ci.

11. Ensemble d'accès (100 ; 200 ; 300 ; 400) selon n'importe quelle revendication précédente, dans lequel la structure anti-effondrement (140 ; 340) est formée d'une matière ayant un profil en coupe transversale circulaire, triangulaire, rectangulaire ou ovale.
